# EUROPEAN PATENT SPECIFICATION

(11) **EP 1 783 127 B1**
(45) Date of publication and mention of the grant of the patent: **30.03.2016**
(21) Application number: 05751348.3
(22) Date of filing: 20.06.2005
(51) Int. Cl.: A23L 33/10, A23L 2/52, C07D 407/14

(54) **Polyphenols from Oolong tea having inhibitory effect on lipase**
Aus Oolong Tee stammende Polyphenole mit Lipase hemmendem Effekt
Polyphénols du thé Oolong ayant une activité inhibitrice sur la lipase

(30) Priority: 21.06.2004 JP 2004182471
(43) Date of publication of application: 09.05.2007
(73) Proprietor: Suntory Holdings Limited, Kita-ku, Osaka-shi Osaka 530-8203 (JP)
(72) Inventor: NAKAI, Masaaki, 5620041 (JP); FUKUI, Yuko, 5691123 (JP); ASAMI, Sumio, 5670886 (JP)
(74) Representative: Vossius & Partner Patentanwälte Rechtsanwälte mbB
(86) International application number: PCT/JP2005/011258
(87) International publication number: WO 2005/123725

(56) References cited:
- -A-JP2000 226 329
- JP-A- 2000 226 329
- NAKAI MASAAKI ET AL: "Inhibitory effects of oolong tea polyphenols on pancreatic lipase in vitro" JOURNAL OF AGRICULTURAL AND FOOD CHEMISTRY, AMERICAN CHEMICAL SOCIETY, SCIENTIFIC PUBLISHERS, NEW DELHI - INDIA LNKD- DOI:10.1021/JF047814+, vol. 53, no. 11, 1 June 2005 (2005-06-01), pages 4593-4598, XP002417716 ISSN: 0021-8561
- ASHIMOTO H OKALA N. AND ISHIOKA N. ET AL.: 'Tannins and related compounds. LXIX. Isolation and structure elucidation ofB.B´-linked bisflavanoids, theasinensins D-G and oolongtheanin from oolong tea. (2),' CHEMICAL PHARMACEUTICAL BULLETIN vol. 36, no. 5, 1988, pages 1676 - 1684, XP002991752
- ANAKA T. ET AL.: 'Production of theasinensins A and D, epigallocatechin dimers of black tea, by oxidation-reduction distutation of dehydrotheasinensin A.' TETRAHEDRON vol. 59, no. 40, 2003, pages 7939 - 7947, XP004458542
- ASHIMOTO H. ANAKA G. AND ISHIOKA N. ET AL.: 'Tannis and related compounds. CXI. Structures of novel fermentation products, theogallinin, theaflavonin and desgalloyl theaflavonin from black tea, and changes of tea leaf polyphenols during fermentation.' CHEMICAL PHARMACEUTICAL BULLETIN vol. 40, no. 6, 1992, pages 1383 - 1389, XP002991753
- SANG S. ET AL.: 'Theadibenzotropolone A, a new type pigment from enzymatic oxidation of (-)-epicatechin and (-)-epigallocatechin gallate and characterized from black tea using LC/MS/MS.' TETRAHEDRON LETTERS vol. 43, no. 40, 2002, pages 7129 - 7133, XP004385602
- SANG S. ET AL.: 'New dibenzotropolne derivativcs characterized from black tea using LC/MS/MS.' BIOORGANIC & MEDICINAL CHEMISTRY. vol. 12, no. 11, 01 June 2004, pages 3009 - 3017, XP002991754
- ASHIMOTO H OKALA N AND ISHIOKA N ET AL: 'Tannins and related compounds. LXIX. Isolation and structure elucidation ofB.B'-linked bisflavanoids, theasinensins D-G and oolongtheanin from oolong tea. (2)' CHEMICAL AND PHARMACEUTICAL BULLETIN vol. 36, no. 5, 01 January 1988, PHARMACEUTICAL SOCIETY OF JAPAN, TOKYO, JP, pages 1676 - 1684, XP002991752 ISSN: 0009-2363
- TANAKA T ET AL: "Production of theasinensins A and D, epigallocatechin gallate dimers of black tea, by oxidation-reduction dismutation of dehydrotheasinensin A", TETRAHEDRON, ELSEVIER SCIENCE PUBLISHERS, AMSTERDAM, NL, vol. 59, no. 40, 29 September 2003 (2003-09-29), pages 7939-7947, XP004458542, ISSN: 0040-4020, DOI: DOI:10.1016/J.TET.2003.08.025
- ASHIMOTO H ANAKA G AND ISHIOKA N ET AL: "Tannis and related compounds. CXI. Structures of novel fermentation products, theogallinin, theaflavonin and desgalloyl theaflavonin from black tea, and changes of tea leaf polyphenols during fermentation", CHEMICAL AND PHARMACEUTICAL BULLETIN, PHARMACEUTICAL SOCIETY OF JAPAN, TOKYO, JP, vol. 40, no. 6, 1 January 1992 (1992-01-01), pages 1383-1389, XP002991753, ISSN: 0009-2363
- SANG S ET AL: "Theadibenzotropolone A, a new type pigment from enzymatic oxidation of (-)-epicatechin and (-)-epigallocatechin gallate and characterized from black tea using LC/MS/MS", TETRAHEDRON LETTERS, ELSEVIER, AMSTERDAM, NL, vol. 43, no. 40, 30 September 2002 (2002-09-30), pages 7129-7133, XP004385602, ISSN: 0040-4039, DOI: DOI:10.1016/S0040-4039(02)01707-0
- SANG S ET AL: "New dibenzotropolne derivativcs characterized from black tea using LC/MS/MS", BIOORGANIC & MEDICINAL CHEMISTRY, PERGAMON, GB, vol. 12, no. 11, 1 June 2004 (2004-06-01), pages 3009-3017, XP002991754, ISSN: 0968-0896, DOI: DOI:10.1016/J.BMC.2004.03.027
- IWATA K: "Shishitsu Taisha ni Oyobosu Oolong-cha no Eikyo. /EFFECS OF OOLONG TEA EN LIPED METABOLESM", JOSHI EIYO DAIGAKU KIYO - JOURNAL OF KAGAWA NUTRITION COLLEGE, KAGAWA EIYO GAKUEN, TOKYO, JP, vol. 27, 1 January 1996 (1996-01-01), pages 11-21, XP002991871, ISSN: 0286-0511
- HARA Y ET AL: "Polyphenol Kyoka Oolongcha Sesshu ni yoru Shibo Sesshugo no Kessei Triglyceride Josho Yokusei Koka. /SUPPRESSIVE EFFECT OF OOLONG TEA POLYMERIZED POLYPHENOLS-RNRICHED OOLONG TEA ON POSTPRANDIAL SERUM TRIGLYCERIDE ELEVATION", YAKURI TO CHIRYO - JAPANESE PHARMACOLOGY AND THERAPEUTICS, RAIFU SAIENSU SHUPPAN, TOKYO, JP, vol. 32, no. 6, 20 June 2004 (2004-06-20), pages 335-342, XP002991869, ISSN: 0386-3603
- GRIFFITHS ET AL: "The inhibition of digestive enzymes by polyphenolic compounds", ADVANCES IN EXPERIMENTAL MEDICINE AND BIOLOGY, SPRINGER, US, vol. 199, 1 January 1986 (1986-01-01), pages 509-516, XP008135219, ISSN: 0065-2598

## Description

### TECHNICAL FIELD

This invention provides novel uses of a polyphenol extracted from oolong tea having lipase inhibitory activity, which may be provided in the form of foods and/or beverages, and pharmaceutical compositions containing the polyphenol for use in the prevention or treatment of obesity, as further defined in the claims.

### BACKGROUND ART

With the recent tendency toward westernized eating habits in Japan, intake of high fat diet continues to increase. According to a National Nutrition Survey in Japan (1999) among people over 60, it is reported that although their energy intake is decreasing every year, their fat energy ratio exceeds the reasonable proportion of 25%, and 50 to 60% of those people are recognized to have high triglyceride and cholesterol values [A Summary of 1999 National Nutrition Survey in Japan by The Ministry of Health, Labor and Welfare, Rinsho Eiyo (Clinical nutrition) 2001; 98(5): 577-588].

Obesity is one of the most severe diseases in present day society, caused by excessive fat intake. The excessive fat intake causes not only obesity, but also contracting disorders such as diabetes, hyperlipidemia, hypertension and arteriosclerosis. In Japan, Mazindole (registered trademark) as an anorectic drug is only one therapeutic drug with official approval for treating obesity. However, this drug is reported to have side effects such as excessive thirst (mouth dryness), constipation, epigastric distress, nausea and vomiting [Rinsyo Hyouka (Clinical evaluation), 1985; 13(2): 419-459, Clinical evaluation, 1985; 13(2): 461-515]. In overseas, Xenical (registered trademark) as a lipase inhibitor which suppresses fat absorption in the gastrointestinal tract, is on market as an obesity treatment drug. However, this drug is also reported to have side effects such as fatty stool, increased stool frequency, soft stool, diarrhea and stomachache. Therefore, using this drug is sometimes accompanied by concerns about safety (The Lancet 1998; 352:67-172) .

To prevent obesity, it is advantageous to reduce the caloric intake by controlling diet. However, it requires careful guidance on nutrition making it difficult to practice in daily life. Therefore, inhibiting the absorption of dietary lipids in the body in a safe and healthy manner is practical and useful for treatment of obesity and related diseases and in promoting health.

With these facts in mind, the development of a "food for specified health uses" which is safe to use and is proven to be effective in treating humans is attracting a lot of attention. Food materials which inhibit increase of serum triglyceride after a meal, such as: a globin protein decomposition product that suppresses fat absorption by pancreatic lipase inhibitory activity [J. Nutr. 1988; 128: 56-60, 1988, Nihon Eiyou Shokuryou Gakkai-shi (Journal of Japanese society of Nutrition and Food Science) 1999; 52(2): 71-77, Kenkou Eiyou Shokuhin Kenkyu (Health food and nutrition food Research) 2002; 5(3): 131-144]; diacylglycerol with different digestion and absorption features compared to triacylglycerol (J. Am. Coll. Nutr. 2000; 19(6): 789-796, Clin. Chim. Acta. 2001; 11(2): 109-117); eicosapentaenoic acid (EPA) and docosahexanoic acid (DHA) purified from fish oil; are on market as foods for specified health use until now.

On the other hand, lipase inhibitors derived from plants are also attracting attention in recent years. Especially, as to the polyphenols with lipase inhibitory activity, for example: tannin from bark (JP Shou 60-11912-B); tannins, flavonoids and glucosides thereof from leguminous plant (Cassia mimosoides L.var.nomame Makino) (JP Hei 8-259557-A); epigallocatechin gallate which is the main component in green tea, and lipid absorption suppressing food containing the epigallocatechin gallate (JP Hei 3-228664-A); lipase inhibitory agent containing water extracts from green pepper, shimeji mushrooms, pumpkin, Grifola frondosa (maitake), Hizikia fusiforme, green tea, oolong tea, and others (JP Hei 3-219872-A); flavons and flavonols (JP Hei 7-61927-A); hydroxybenzoic acids (gallic acid) (JP Hei 1-102022-A); triterpenes and derivatives thereof (JP Hei 9-40689-A) ; and anti-obesity medicine containing procyanidin from Tamarind as an active ingredient (JP Hei 9-291039-A) are reported.
Also, the lipase inhibitory effect of grape seed extract (Nutrition vol.19, (10), 876-879,2003), the lipase inhibitory effect and anti-obesity effect of polyphenol from Salacia in rats (J. Nutr., 132, 1819-1824, 2002), anti-obesity effect of oolong tea extract in rats (Int. J. Obes., 23 98-105, 1999), and others are known.

However, reported lipase inhibitory agents from plants mentioned above are not sufficiently effective. For instance, since they originate from natural sources, there is a problem in maintaining stable lipase inhibitory activity when the content of the active ingredient in the plant is not clearly known. Moreover, an inhibitory agent derived from plants with less preference will raise a flavor problem when used in foods and/or beverages. For example, reports on lipid-improving effect of oolong tea are: significant decrease in blood triglyceride after drinking 1330ml/day of commercial oolong tea for 6 weeks [Nihon Eiyou Shokuryou Gakkai-shi (Journal of Japanese society of Nutrition and food science) 1991; 44(4): 251-259]; and oral administration of oolong tea (2 g x 4/day) for 6 consecutive weeks to 102 males and females with simple obesity resulted in more than 1 kg weight loss in 67% of the subjects and significant improvement in the subjects with high blood acylglycerol after taking oolong tea [Nihon Rinsho Eiyou Gakkai-shi (The Japanese Society of Clinical Nutrition Magazine) 1998; 20(1): 83-90]. These reports show that although drinking a large quantity of oolong tea is recognized to be effective, it is difficult in daily life to continue drinking such large quantities of a drink such as oolong tea. Further, simply providing concentrated oolong tea is not an appropriate and a practical option, due to its strong bitterness and astringency and increased caffeine content.
JP 2000-226329 A relates to an MMPs(matrix metaprotease) inhibitor containing a catechin compound which can be afforded by subjecting tea leaves including green tea and black tea leaves to extraction with an aqueous solvent such as water or a lower alcohol or an organic solvent such as acetone or ethyl acetate, or by extraction and isolation from other plant(s), or by chemical synthesis, being e.g. theasinensin A, theasinensin F, theasinensin D, theasinensin G, oolongtheanin. Chem. Pharm. Bull. 1988, 36(5): 1676-1684 describes the isolation of theasinensins D-G, a dimeric flavan-3-ol named oolong-theanin, and theasinensins A, B, and C.
Tetrahedron 2003, 59(40): 7939-7947 discloses the production of theasinensins A and D by oxidation-reduction dismutation of dehydrotheasinensin A.
Chem. Pharm. Bull. 1992, 40(6): 1383-1389 describes the isolation of the fermentation products theogallinin, theaflavonin and desgalloyl theaflavonin from black tea. Tetrahedron Letters 2002, 43(40): 7129-7133 discloses the formation of theadibenzotropolone A and theaflavin 3-gallate by the reaction of (-)-epicatechin and (-)-epigallocatechin gallate with horseradish peroxidase in the presence of H₂O₂. Bioorg. Med. Chem. 2004, 12(11): 3009-3017 describes the formation of theadibenzotropolone A, B, and C as well as theatribenzotropolone A by the reaction of theaflavins and tea catechins with horseradish peroxidase in the presence of H₂O₂. Journal of Kagawa Nutrition College 1996, 27: 11-21 relates to effects of oolong tea on lipid metabolism.
Jpn. Pharmacol. Ther. 2004, 32(6): 335-342 describes an evaluation of the suppressive effect of OTPP (oolong tea polymerized polyphenols) enriched oolong tea beverage on postprandial serum triglyceride elevation.
Adv. Exp. Med. Biol. 1986, 199: 509-16 relates to the inhibition of digestive enzymes by polyphenolic compounds.

### DISCLOSURE OF THE INVENTION

### PROBLEM TO BE SOLVED BY THE INVENTION

The present invention targets particular components in tea that has high preference, and provides novel uses of a polyphenol present in oolong tea having lipase inhibitory activity, as defined in the claims.

The present invention further provides uses of the polyphenol compound of the invention having lipase inhibitory activity provided in the form of foods and/or beverages, which will suppress the absorption of dietary lipids and suppress the rise of triglyceride in blood.

The present invention further provides a pharmaceutical composition containing the polyphenol compound of the invention having lipase inhibitory activity for use in the prevention or treatment of obesity.

### MEANS FOR SOLVING PROBLEM

The inventors found that oxidative polymerization of epigallocatechin-3-O-gallate with a tea leaf enzyme (polyphenol oxidase) gives a dimer compound, oolongtheanin-3'-O-gallate of the following formula:

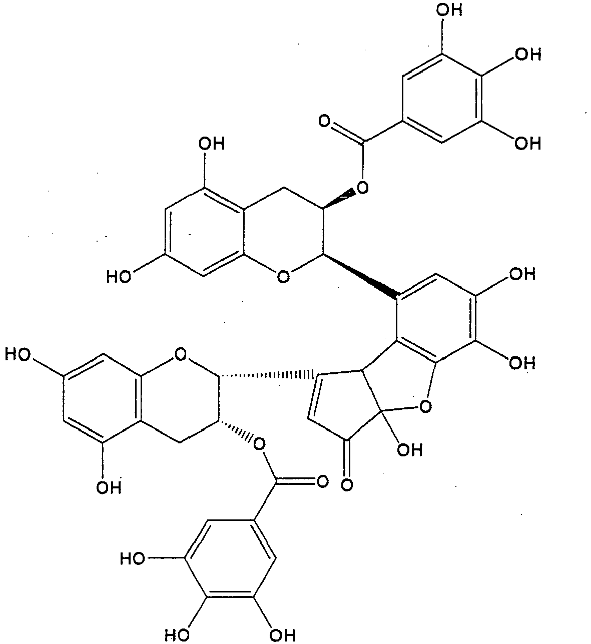

which has a strong inhibitory effect on pancreatic lipase, an enzyme essential for fat absorption.

### Process for preparation

The compound to be used in accordance with the present invention can be obtained by oxidative polymerization of epigallocatechin-3-O-gallate with polyphenol oxidase. Starting material epigallocatechin-3-O-gallate is a known compound which is commercially available. Also, it can be obtained from natural materials such as green tea, black tea, and oolong tea by extraction. Polyphenol oxidase used for oxidative polymerization can be prepared by, for example, extraction from tealeaf according to the process described in Example 1. However, it is not limited to enzymes of tealeaf origin as long as it catalyzes the oxidative polymerization reaction of epigallocatechin-3-O-gallate into oolongtheanin-3'-O-gallate (OTNG). For example, an enzyme derived from horseradish can be used.

The oxidative polymerization reaction is carried out by placing a starting material (epigallocatechin-3-O-gallate), an oxidiant (for example, H₂O₂), and polyphenol oxidase in an aqueous buffer solution with pH 4 to 7, preferably pH 5 to 6, at 20 to 40°C, preferably 25 to 35°C, for 1 to 4 hours, preferably 3 hours. For 100 mg of starting material, for example, 2 mg of oxidant and polyphenol oxidase obtained from 100 g of fresh tealeaf, can be used respectively.

The product obtained from oxidative polymerization reaction may be purified by conventional methods such as chromatography. Purified oolongtheanin-3'-O-gallate is a "white powder, soluble in water, methanol and DMSO, and neutral", which is very safe. Therefore, this compound is suitable for use in, for example, foods and/or beverages, and pharmaceutical drugs as an active lipase inhibitory ingredient for suppressing absorption of dietary lipids, thereby suppressing the rise of blood triglyceride or decreasing increased blood triglyceride.

Although oolongtheanin-3'-O-gallate is provided as a result of the above oxidative polymerization reaction, there is a possibility that it is present in natural materials such as tealeaves. Therefore, the compound may be obtained from those natural materials by extraction and purification.

### Method for lipase inhibitory activity determination

The compound to be used in accordance with the present invention has strong inhibitory activity against lipase, particularly pancreatic lipase. This inhibitory activity can be determined by the method specifically described in Example 2.

### Lipase inhibitor

The compound to be used in accordance with the present invention may be used as a lipase inhibitor either alone or with a solvent or a solid carrier. Preferably, the solvent or carrier is safe to use in foods or medicament, considering its use in foods and/or beverages and/or medicament mentioned below. The lipase inhibitor can be used for various purposes, including, for example, experimental purposes, and as an active ingredient of preventing accumulation of triglyceride in foods and medicaments.

### Foods and/or beverages containing oolongtheanin-3'-O-gallate

The compound to be used in accordance with the invention or the lipase inhibitory agent containing the compound may be added to foods and/or beverages as an active lipase inhibitory ingredient in order to prevent undesirable rise of blood triglyceride that may accompany fat intake from diet, and/or to decrease increased blood triglyceride levels. Preferable examples of foods and/or beverages are daily taken foods and/or beverages, such as green tea, mugi-cha (barley tea), oolong tea, black tea, coffee, sports drink, drinking water, seasoning and dressing. However, the foods and/or beverages may be any of those taken usually, such as a soft drink, cocktail, beer, whisky, shochu (rough distilled spirits), wine, sake, seasoning, dressing, flavored rice, processed food, instant food, retort pouch (specially packaged food that has been pre-heated and sterilized), chocolate, fresh cream, confectionery, dairy products (nyu-seihin), health foods, and dietary supplements.

The compound to be used in accordance with the present invention is added to foods and/or beverages to provide 0.1 mg to 1000 mg of intake per meal. Provided that since the compound to be used in accordance with the present invention derives from food, it is very safe, and there is no practical upper limit of the amount which can be added to foods and/or beverages.

### A pharmaceutical drug containing oolongtheanin-3'-O-gallate

The compound to be used in accordance with the present invention can also be used as an active ingredient in a drug for use in preventing or treating obesity. Preferable drugs are drugs suitable for oral administration, such as drinks, tablets, capsules, granules, powders, candies and drops. The drugs comprise the compound to be used in accordance with the present invention in amounts of 0.1 mg to 1000 mg per dose.

Since the active lipase inhibitory ingredient oolongtheanin-3'-O-gallate is very safe, the pharmaceutical drug of the present invention can be administered for a long time without the risk of side effects. Therefore, it may be taken daily for the purpose of preventing or treating obesity as a life-style disease.

### EFFECT OF THE INVENTION

By adding polyphenol derived from oolong tea, the present invention provides foods and/or beverages of high preference for reducing triglyceride and promoting health without spoiling flavor. In order to inhibit absorption of dietary lipids, it is desirable to take it with meal. Therefore, beverages enriched with the active ingredient obtained from tea are highly significant.

The compound to be used in accordance with the present invention is prepared by a simple process with epigallocatechin-3-O-gallate as a starting material, which is present in large amounts in oolong tea. The purification process is also easy.

### BRIEF DESCRIPTION OF THE DRAWINGS

Fig. 1 shows MS spectrum of Oolongtheanin-3'-O-gallate (OTNG).
Fig. 2 shows ¹H NMR data of OTNG.
Fig. 3 shows ¹³C NMR data of OTNG.
Fig. 4 shows chemical structure of OTNG.

### Examples

### Example 1 Enzymatic synthesis of oolongtheanin-3'-O-gallate (OTNG)

### Enzyme preparation

600 g of Tealeaves, Kyoken No. 129 (provided from Kyoto Prefectural Tea Industry Research Institute) was triturated in liquid nitrogen. 1800 ml of extraction buffer (adjusted to pH7.0 with 0.01M KH₂PO₄ and 0.02M K₂HPO₄) and 300g of polyamide were added and stirred, then filtered through gauze. The filtrate was centrifuged for 20 minutes at 8000 rpm. 1500 ml of acetone cooled to -20°C in advance was added to 1500 ml of the supernatant, and the mixture was left to stand at 4°C for 1 hour. The solution was centrifuged at 8000 rpm for 20 minutes at 4°C, to obtain a white precipitate. The precipitate was dissolved in 600 ml of a reaction buffer (adjusted to pH 5.6 with 0.01M citric acid and 0.02M KH₂PO₄) to obtain an enzyme solution.

### Enzyme reaction

600 mg of epigallocatechin-3-O-gallate (Wako Pure Chemical Industries, Ltd.) and 8.8 mM of H₂O₂ were added to 600ml of the enzyme solution. After stirring, the reaction took place at 32°C. After 3 hours, 600 ml of 90% acetonitrile containing 1% trifluoroacetic acid (TFA) was added to terminate the reaction. The solution was diluted 5-fold with water and applied to absorption resin HP-20 (1000 ml, Mitsubishi Chemical Corporation). After washing with water, the reaction product was eluted with 2000 ml of 90% acetonitrile containing 0.1% TFA. The reaction product was concentrated under reduced pressure, and then lyophilized.
The lyophilized product was purified by the following preparative HPLC.

### Purification

Column: Develosil ODS-UG-5 (50mmφ x 500 mm, Nomura Chemical)
Mobile phase: A: 0.05%TFA/H₂O, B:90%CH₃CN, 0.05%TFA
Detection: A280 nm
Flow rate: 32 ml/min
Gradient: linear gradient elution from B20% to B50% for 100 min.

Oolongtheanin-3'-O-gallate was derived by chromatography and at an elution time of 52 minutes. Another preparative HPLC was carried out for further purification. Column: Develosil C30-UG-5 (20 mmφ x 250 mm, Nomura Chemical) Mobile phase: A : 0.1%TFA/H₂O, B : 90%CH₃CN, 0.1%TFA
Detection: A280 nm
Flow rate: 6 ml/min
Gradient: linear gradient elution from B10% to B40% for 40 min.

This chromatography gave 25mg of oolongtheanin-3'-O-gallate at an elution time of 34 minutes.

The isolation of oolongtheanin (no gallate) from tealeaves has been reported in Chem.Pharm.Bull 36(5), 1676-1684, 1988. However, oolongtheanin-3'-O-gallate is obtained in Example 1.

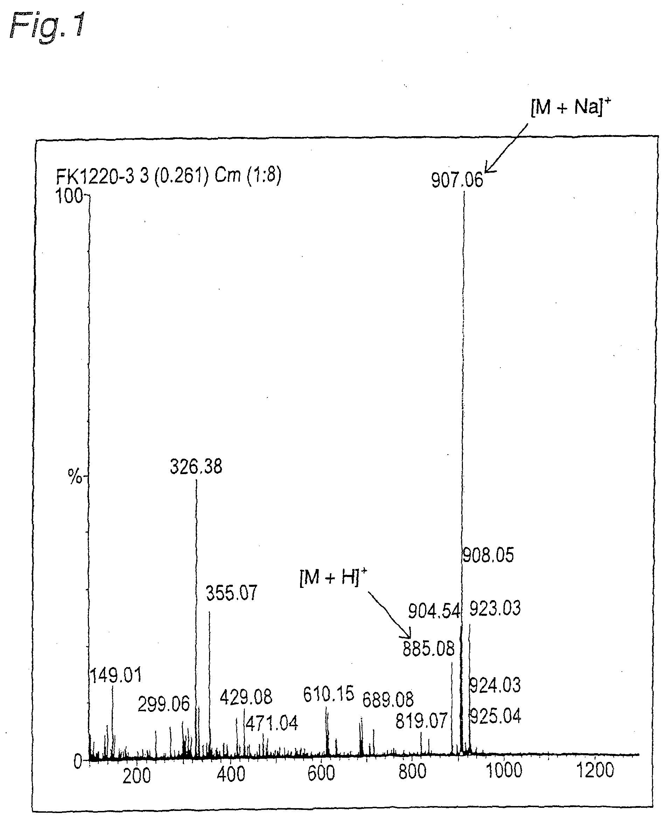

MS was measured with Q-TOF (Micromass, Manchester, UK) using ESI probe, in a positive mode. Ion peaks were observed at m/z 885 for [M+H]⁺ and at m/z 907 for [M+Na]⁺. The spectrum is shown in Fig. 1.

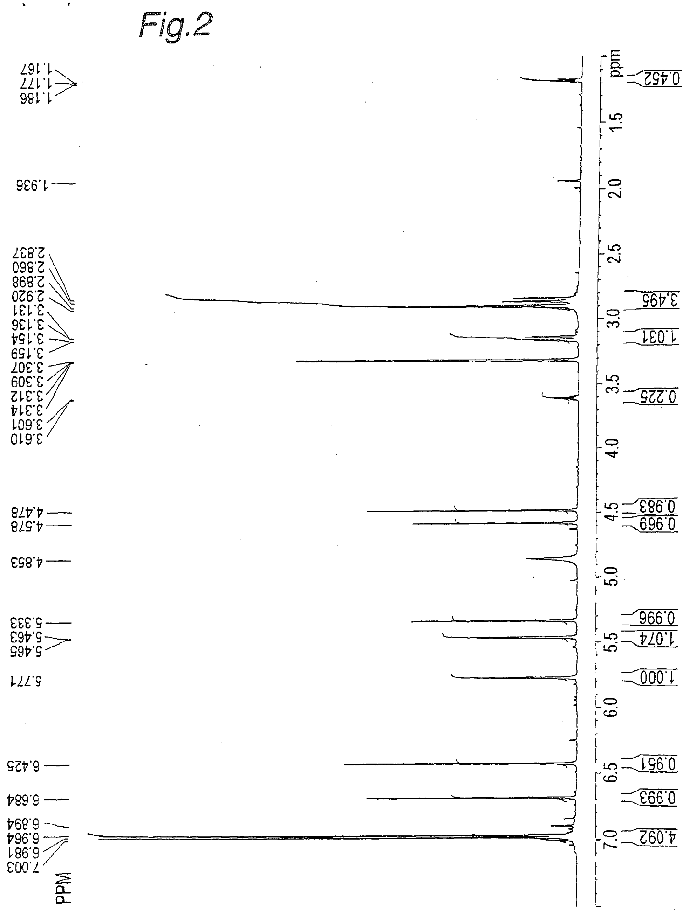

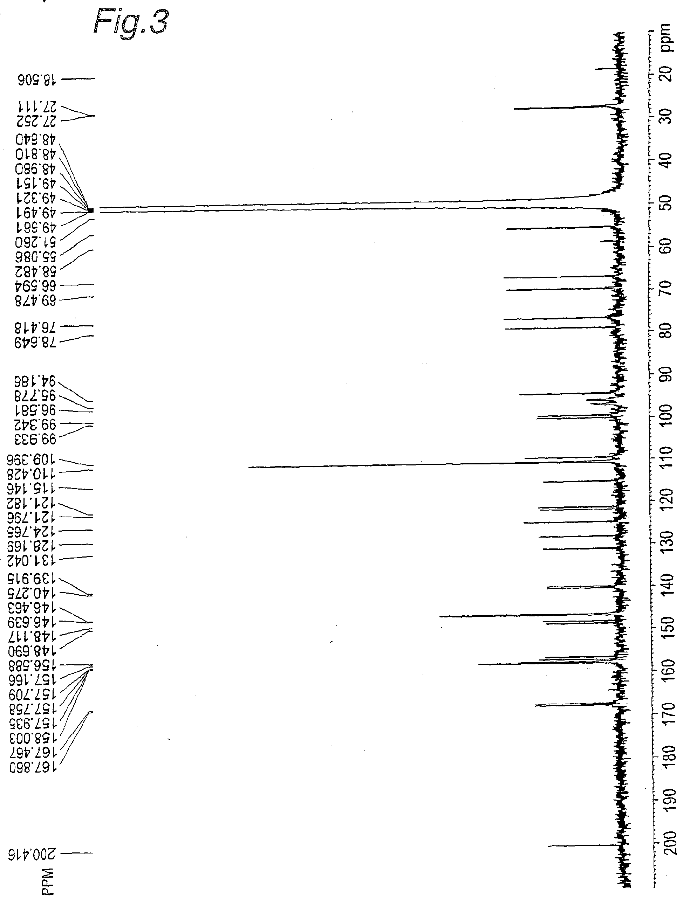

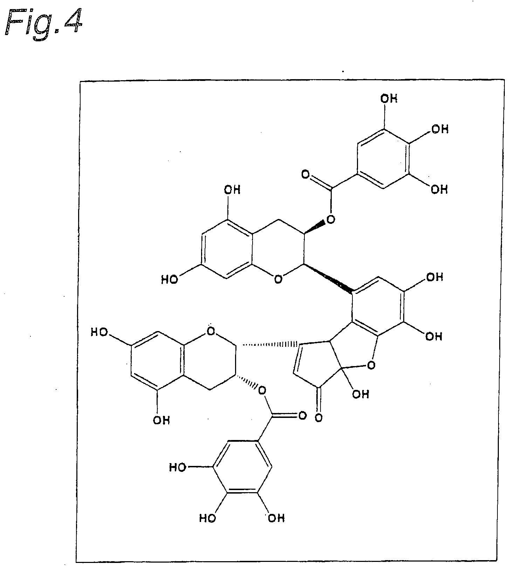

¹H NMR, ¹³C NMR, ¹H{¹³C}-HSQC, ¹H{¹³C}-HMBC, TOCSY, and DQF-COSY were measured with DMX-750 (BRUKER BIOSPIN) in CD₃OD. ¹H NMR and ¹³C NMR are shown in Figs. 2 and 3, respectively. The structural formula is shown in Fig. 4.

### Example 2 Lipase inhibitory activity measurement

Lipase activity measurement was carried out by using oleic acid ester of fluorescent 4-methylumbelliferone (4-UMO) as a substrate, and measuring the fluorescence of 4-methylumbelliferone produced by reaction.

In the measurement, 13 mM Tris-HCl containing 150 mM NaCl and 1.36mM CaCl₂ was used as a buffer (pH 8.0).
Substrate 4-UMO (Sigma) was prepared as 0.1M solution in DMSO and diluted 1000-fold with the buffer mentioned above. Similarly, lipase (porcine pancreatic lipase (Sigma)) was prepared as 400 U/ml solution in the buffer mentioned above and used in enzymatic measurement.

50 µl of the 4-UMO buffer solution and 25 µl of distilled water (or sample solution) were placed in a 96-well microplate and mixed at 25°C, followed by adding 25 µl of the lipase buffer solution to start enzyme reaction. After 30 minutes of reaction, 100 µl of 0.1M citric acid buffer (pH 4.2) was added to terminate the reaction, and the fluorescence of 4-methylumbelliferone (excitation wavelength: 355 nm, fluorescence wavelength: 460 nm) produced by the reaction was measured with a fluorescence plate reader (Labsystems, Fluoroskan Asent CF).

An inhibitory activity of the sample was determined as IC₅₀, or the amount of the sample which gave 50% of inhibition compared to the activity of control (distilled water). As to the lipase inhibitory activity of OTNG, its IC₅₀ was 0.06 µg/ml (0.068 µM), showing it to be extremely high in activity compared to EGCG monomer whose IC₅₀ was 0.16 µg/ml (0.349 µM).

## Claims

1. Use of oolongtheanin-3'-O-gallate having the formula (I): 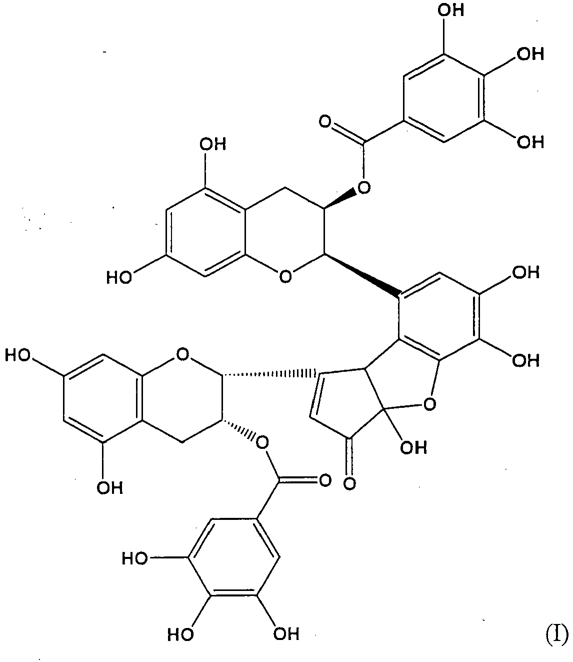 for suppressing absorption of dietary lipids; and/or
for suppressing the rise of triglycerides and/or for decreasing increased triglyceride levels in blood;
with the proviso that the use in methods for treatment of the human or animal body by therapy is excluded.

2. The use according to claim 1, wherein the oolongtheanin-3'-O-gallate having the formula (I) is provided in the form of a food or beverage.

3. The use according to claim 2, wherein the food or beverage is selected from the group consisting of tea beverages, soft drinks and health foods.

4. The use according to claim 2 or 3, wherein the oolongtheanin-3'-O-gallate having the formula (I) is present in the food or beverage in an amount so as to provide 0.1 mg to 1000 mg of intake per meal.

5. A pharmaceutical composition comprising oolongtheanin-3'-O-gallate having the formula (I): 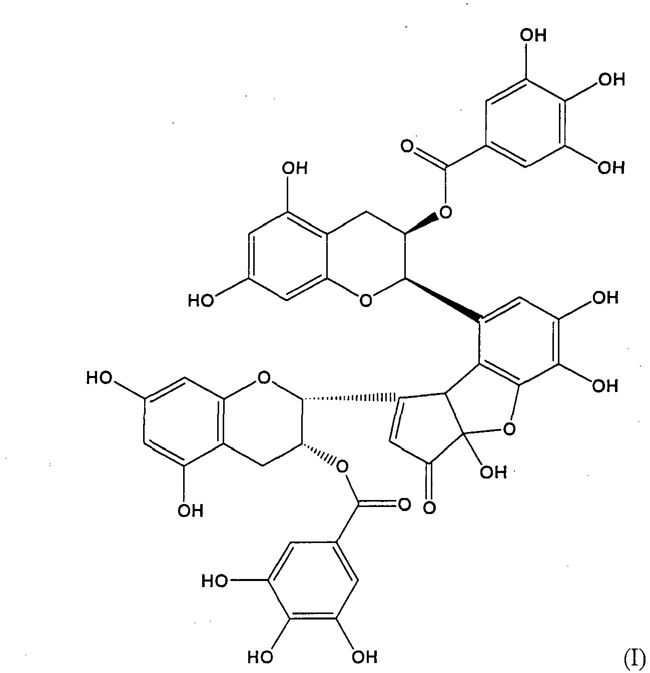 for use in the prevention or treatment of obesity.

6. The pharmaceutical composition according to claim 5, wherein the oolongtheanin-3'-O-gallate having the formula (I) is to be administered in an amount of 0.1 mg to 1000 mg per dose.

7. Use of oolongtheanin-3'-O-gallate having the formula (I): 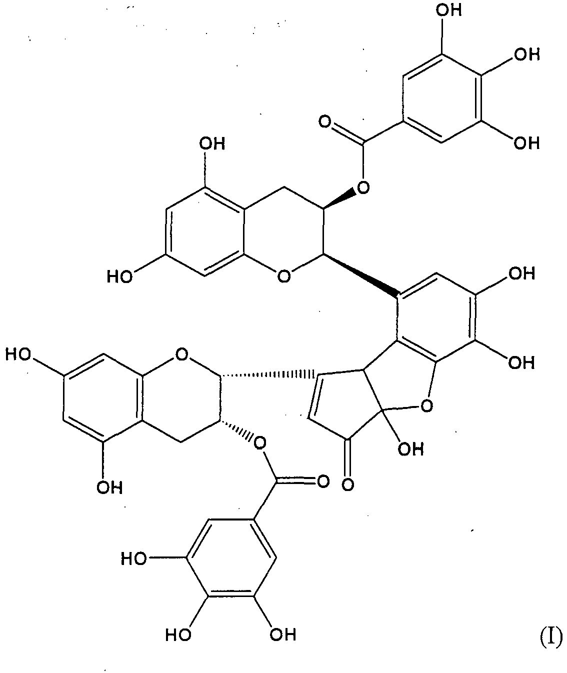 for the preparation of a medicament for preventing or treating obesity.

8. The use according to claim 7, wherein the oolongtheanin-3'-O-gallate having the formula (I) is to be administered in an amount of 0.1 mg to 1000 mg per dose.

## Patentansprüche

1. Verwendung von Oolongtheanin-3'-O-gallat, das die Formel (I) aufweist: 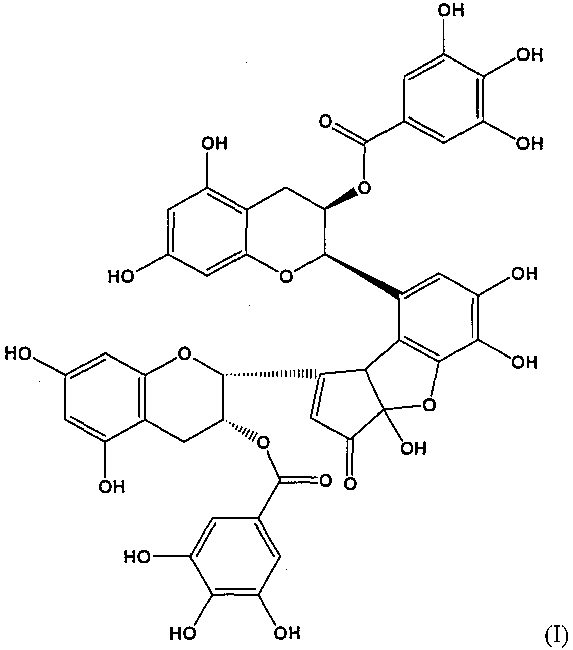 zur Unterdrückung der Aufnahme von Nahrungsfetten; und/oder
zur Unterdrückung des Anstiegs von Triglyceriden und/oder zur Verringerung von erhöhten Triglyceridspiegeln im Blut;
mit der Maßgabe, dass die Verwendung in Verfahren zur therapeutischen Behandlung des menschlichen oder tierischen Körpers ausgeschlossen ist.

2. Die Verwendung gemäß Anspruch 1, wobei das Oolongtheanin-3'-O-gallat, das die Formel (I) aufweist, in der Form eines Nahrungsmittels oder Getränks bereitgestellt ist.

3. Die Verwendung gemäß Anspruch 2, wobei das Nahrungsmittel oder Getränk aus der Gruppe bestehend aus Teegetränken, Softdrinks und Gesundheitskost ausgewählt ist.

4. Die Verwendung gemäß Anspruch 2 oder 3, wobei das Oolongtheanin-3'-O-gallat, das die Formel (I) aufweist, in dem Nahrungsmittel oder Getränk in einer Menge vorhanden ist, um 0,1 mg bis 1000 mg Aufnahme pro Mahlzeit bereitzustellen.

5. Ein Arzneimittel, umfassend Oolongtheanin-3'-O-gallat, das die Formel (I) aufweist: 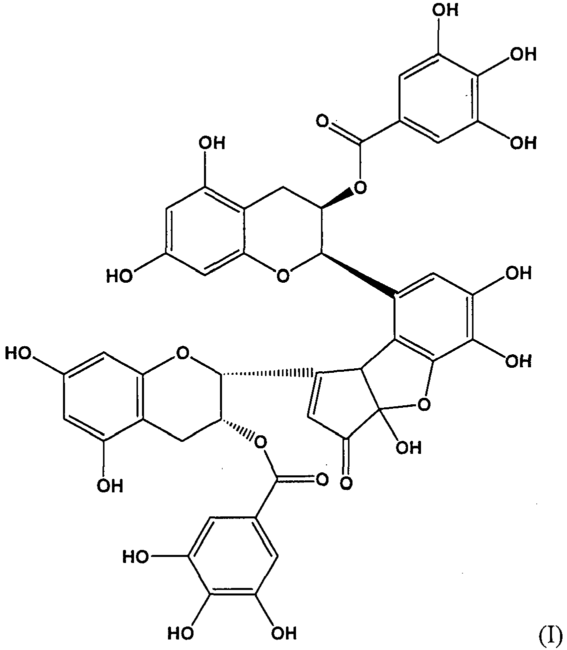 zur Verwendung bei der Vorbeugung oder Behandlung von Fettleibigkeit.

6. Das Arzneimittel gemäß Anspruch 5, wobei das Oolongtheanin-3'-O-gallat, das die Formel (I) aufweist, in einer Menge von 0,1 mg bis 1000 mg pro Dosis verabreicht werden soll.

7. Verwendung von Oolongtheanin-3'-O-gallat, das die Formel (I) aufweist: 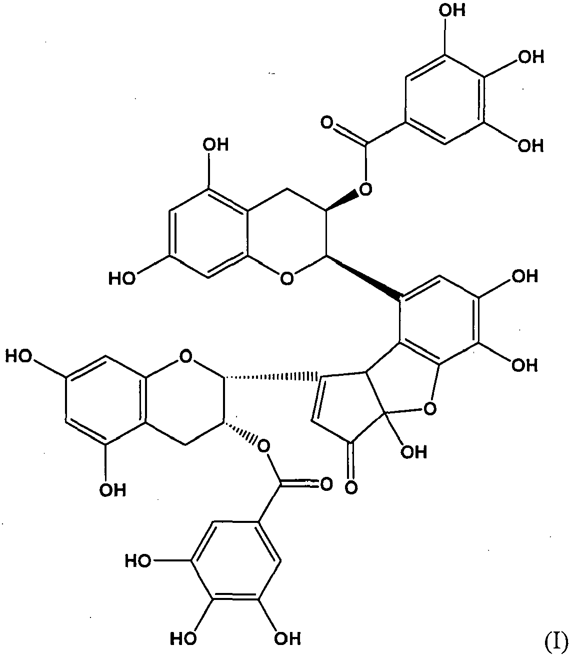 für die Herstellung eines Medikaments zur Vorbeugung oder Behandlung von Fettleibigkeit.

8. Die Verwendung gemäß Anspruch 7, wobei das Oolongtheanin-3'-O-gallat, das die Formel (I) aufweist, in einer Menge von 0,1 mg bis 1000 mg pro Dosis verabreicht werden soll.

## Revendications

1. Utilisation d'oolongthéanine-3'-O-gallate répondant à la formule (I) : 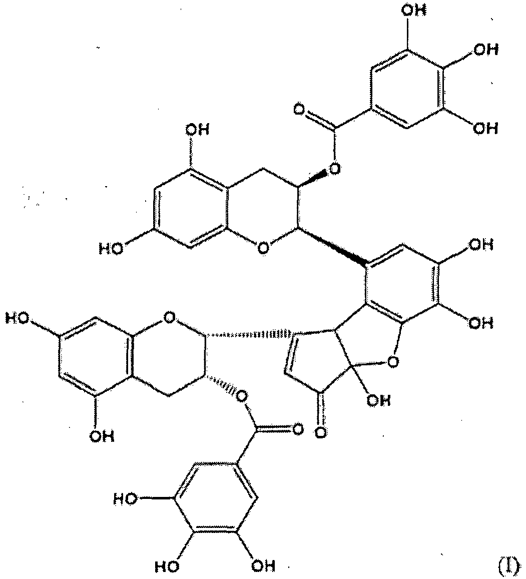 pour la suppression de l'absorption de lipides alimentaires ; et/ou
pour la suppression de l'augmentation des triglycérides et/ou pour la diminution de taux de triglycérides accrus dans le sang ;
à condition que l'utilisation dans des méthodes de traitement du corps humain ou animal par thérapie soit exclue.

2. Utilisation selon la revendication 1, dans laquelle l'oolongthéanine-3'-O-gallate répondant à la formule (I) est fourni sous forme d'un aliment ou d'une boisson.

3. Utilisation selon la revendication 2, dans laquelle l'aliment ou la boisson est choisi(e) dans le groupe constitué par les thés, les boissons non alcoolisées et les aliments de santé.

4. Utilisation selon la revendication 2 ou 3, dans laquelle l'oolongthéanine-3'-O-gallate répondant à la formule (I) est présent dans l'aliment ou la boisson en une quantité fournissant de 0,1 mg à 1000 mg d'absorption par repas.

5. Composition pharmaceutique comprenant l'oolongthéanine-3'-O-gallate répondant à la formule (I) : 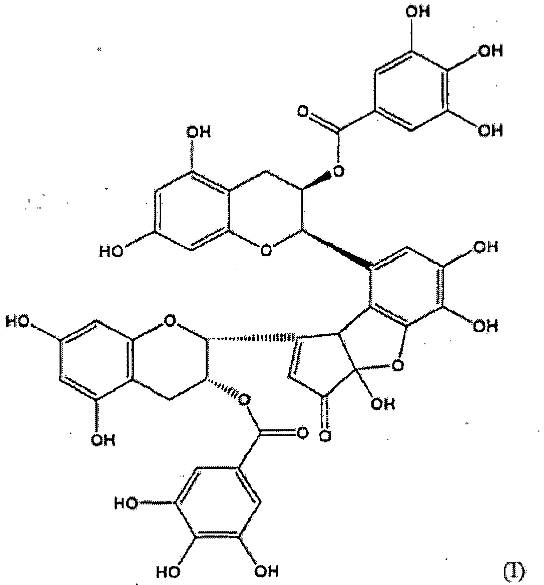 pour une utilisation dans la prévention ou le traitement de l'obésité.

6. Composition pharmaceutique selon la revendication 5, dans laquelle l'oolongthéanine-3'-O-gallate répondant à la formule (I) doit être administré en une quantité de 0,1 mg à 1000 mg par dose.

7. Utilisation d'oolongthéanine-3'-O-gallate répondant à la formule (I) : 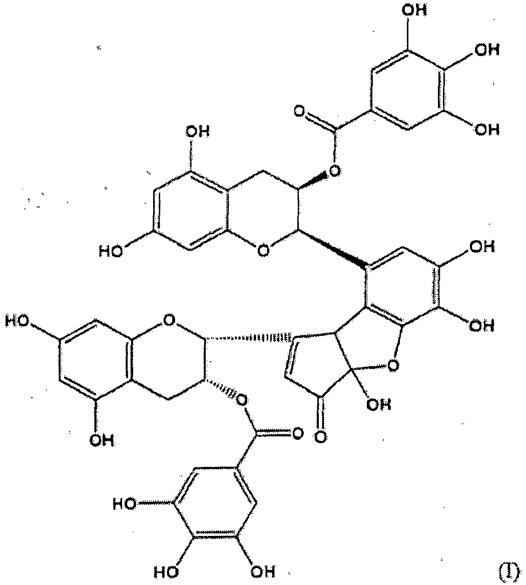 pour la préparation d'un médicament destiné à la prévention ou au traitement de l'obésité.

8. Utilisation selon la revendication 7, dans laquelle l'oolongthéanine-3'-O-gallate répondant à la formule (I) doit être administré en une quantité de 0,1 mg à 1000 mg par dose.
